# FASCICULE DE BREVET EUROPEEN

(11) **EP 4 301 312 B1**
(45) Date de publication et mention de la délivrance du brevet: **24.06.2026**
(21) Numéro de dépôt: 22705553.0
(22) Date de dépôt: 25.02.2022
(51) Int. Cl.: A61H 1/00, A61H 33/00, A61H 21/00, A61H 23/02, A61B 5/07, A61B 5/00

(54) **ENSEMBLE TRANSPORTABLE DE RÉCUPÉRATION MUSCULAIRE PAR IMMERSION**
TRANSPORTABLE ANORDNUNG ZUR MUSKELERHOLUNG DURCH EINTAUCHEN
TRANSPORTABLE ASSEMBLY FOR MUSCULAR RECOVERY BY IMMERSION

(30) Priorité: 01.03.2021 FR 2101954
(43) Date de publication de la demande: 10.01.2024
(73) Titulaire: Cryotank, 33980 Audenge (FR)
(72) Inventeur: PELUCHON, Mathieu, 33980 Audenge (FR); BALTUS, Rudolphus, 33980 Audenge (FR)
(74) Mandataire: Fantin, Laurent
(86) Numéro de dépôt international: PCT/EP2022/054743
(87) Numéro de publication internationale: WO 2022/184559

(56) Documents cités:
- WO-A1-2020/164931
- FR-A1- 2 273 244
- JP-A- S62 290 461
- US-A- 5 269 414
- US-A1- 2005 235 406
- US-A1- 2013 226 044
- US-B1- 6 623 632

## Description

### Domaine technique

La présente invention appartient au domaine des dispositifs paramédicaux et d'entretien du corps humain, notamment les équipements pour la récupération et la stimulation musculaire, et concerne plus particulièrement un ensemble transportable de récupération musculaire par immersion, en eau froide par exemple.

La présente invention trouve une application directe dans la cryothérapie pour sportifs, sans s'y limiter.

### Etat de l'art

La récupération musculaire par immersion dans une eau froide ou contrastée (chaude - froide) est de plus en plus recommandée après des exercices de musculation ou des efforts à forte contrainte énergétique, afin d'accélérer le processus de récupération musculaire.

Cette technique de récupération musculaire, dont les bienfaits ont été prouvés par de nombreuses études scientifiques (Hausswirth C, et al. Réponses physiologiques liées à une immersion en eau froide et à une cryostimulation-cryothérapie en corps entier : effets sur la récupération après un exercice musculaire. Sci sports (2010*)*)*,* est largement répandue, que ce soit auprès d'athlètes de haut niveau ou de sportifs amateurs.

Pour réaliser une récupération musculaire par immersion, il existe des bassins et des cabines destinés à être installés ou construits in situ dans un lieu donné (salle de sport, complexe sportif, centre spécialisé, etc.). Ces bassins ou cabines fixes nécessitent des aménagements spécifiques, voire parfois d'importants travaux.

Le document US2018055685 concerne un système de cryothérapie comprenant un appareil irrigable, qui peut être gonflable, configuré pour s'étendre autour d'une zone de traitement adaptée pour contenir à la fois du cryogène vaporisé et au moins une partie du corps d'un utilisateur, et un système de refroidissement configurable pour fournir du cryogène vaporisé dans la zone de traitement.

Le document KR20200069816 concerne un système de cryothérapie similaire comprenant une chambre pouvant recevoir une personne et fournir un gaz pour la cryothérapie. Le gaz arrive à l'intérieur de la chambre par un tuyau de raccordement sur lequel un moyen de réglage de la pression du gaz est installé.

D'autres solutions plus sophistiquées proposent un suivi automatisé des séances de cryothérapie.

Par exemple, le document US10271986 concerne un système et une méthode pour produire et contrôler automatiquement une séance de cryothérapie dans une chambre, comprenant un système de plomberie pour refroidir la chambre. Un contrôleur central peut être couplé au système de plomberie et utilisé pour : lancer la séance de cryothérapie dans la chambre avec un gaz cryogénique pour refroidir la chambre à une première température ; déterminer si la première température a été atteinte dans la chambre ; déterminer si une commande d'enregistrement a été reçue pour la séance de cryothérapie ; et arrêter la séance de cryothérapie après une période de temps prédéterminée.

Enfin, il existe des bassins de cryothérapie gonflables et transportables. Ces bassins ne sont pas pratiques car nécessitent un temps de gonflage et de remplissage avant chaque utilisation, doivent être dégonflés pour être transportés, et peuvent être facilement endommagés en raison de leur fragilité. De plus, ces bassins ne disposent pas d'un système de filtration.

Dans le domaine des bassins de baignade hors-sol, le document WO2020164931 décrit un bassin comportant un circuit de traitement de l'eau, qui présente une pompe et un filtre, positionné dans l'enveloppe du bassin. Ce bassin de baignade hors-sol est accouplé à une pompe à chaleur pour chauffer l'eau dissimulée dans un châssis en gradin permettant à un utilisateur d'accéder au bassin. La pompe à chaleur est reliée au circuit de traitement de l'eau par des moyens de raccordement permettant de détacher l'ensemble châssis en gradin et pompe à chaleur. Ce bassin n'est pas destiné à être transporté car il ne comprend aucune poignée. De plus, il est relativement lourd dans la mesure où il intègre le circuit de traitement de l'eau. Même si l'ensemble châssis en gradin et pompe à chaleur peut être détaché du bassin, les deux éléments sont relativement volumineux et peuvent difficilement être transportés ou stockés dans un espace restreint.

### Présentation de l'invention

La présente invention vise à pallier les inconvénients de l'art antérieur exposés ci-avant, en proposant une solution pratique, entièrement transportable, ergonomique et peu couteuse, pour être accessible à un large public, et plus particulièrement à des clubs sportifs amateurs ou professionnels avec des moyens limités, des salles de sport, des cabinets paramédicaux, etc. De plus, cette solution ne nécessite aucune modification de son lieu d'utilisation.

À cet effet, la présente invention a pour objet un ensemble de récupération musculaire par immersion selon la revendication 1. Cet ensemble comprend un bassin et une unité fonctionnelle de réfrigération et/ou de chauffage d'un liquide contenu dans le bassin, ladite unité comprenant un groupe froid couplé à un dispositif de filtration et à une pompe en circulation externe, ledit bassin se raccordant à ladite unité par des tuyaux. Cet ensemble est remarquable en ce qu'il est entièrement transportable et en ce que le bassin définit un volume intérieur dans lequel l'unité fonctionnelle peut être rangée.

Ainsi, l'ensemble peut être déployé à différents endroits sans être fixé ou scellé au sol, et rangé de façon peu encombrante en plaçant l'unité fonctionnelle à l'intérieur du bassin lorsque ce dernier a été vidé de son eau, pour être transporté dans le coffre d'un véhicule par exemple.

Le bassin comporte à sa base des semelles espacées, agencées de sorte à dégager des espaces sous le bassin empêchant une stagnation d'eau, permettant un accès en vue d'un nettoyage (aspiration, essuyage, etc.), et protégeant les faces inférieures du bassin lors de son transport.

De plus, les espaces dégagés entre ces semelles permettent de transporter le bassin au moyen d'un transpalette, ce qui est avantageux surtout lorsque le bassin est rempli.

L'ensemble comprend en outre un couvercle de fermeture du bassin pour préserver l'eau du bassin et limiter les échanges thermiques avec l'extérieur. Idéalement, le couvercle assure une fermeture hermétique du bassin.

De façon avantageuse, l'unité fonctionnelle est montée sur un chariot mobile pourvu de roues et/ou de roulettes pour être facilement déplaçable, surtout lorsque l'unité fonctionnelle est destinée à être placée loin du bassin d'immersion.

Selon un mode de réalisation particulier, le bassin et le couvercle sont fabriqués en polyéthylène haute densité (PEHD) pour être à la fois légers et résistants.

Selon un mode de réalisation particulièrement avantageux, le bassin comporte une seule vanne raccordée à l'unité fonctionnelle par un premier tuyau, et l'ensemble comprend un deuxième tuyau raccordé à l'unité fonctionnelle et permettant à la fois de refouler un liquide à température régulée dans le bassin et de vidanger ce dernier.

Ainsi, un embout de refoulement du deuxième tuyau peut être placé loin du bassin pour vidanger ce dernier dans un endroit adapté.

Les concepts fondamentaux de l'invention venant d'être exposés dans leur forme la plus élémentaire, d'autres détails et caractéristiques ressortiront plus clairement à la lecture de la description qui suit et en regard des dessins annexés, donnant à titre d'exemple non limitatif un mode de réalisation d'un ensemble de récupération musculaire par immersion conforme aux principes de l'invention.

### Présentation des dessins

Les figures sont données à titre purement illustratif pour une meilleure compréhension de l'invention sans limiter la portée de celle-ci. Les différents éléments sont représentés de manière schématique et ne sont pas nécessairement à la même échelle. Sur l'ensemble des figures, les éléments identiques ou équivalents portent la même référence numérique.

Il est ainsi illustré en :
- La figure 1 est une vue en perspective d'un ensemble de récupération musculaire par immersion selon un mode de réalisation ;
- La figure 2 est une vue en perspective du bassin de l'ensemble ;
- La figure 3 est une vue en perspective du couvercle du bassin, selon sa face extérieure en (a) et sa face intérieure en (b) ;
- La figure 4 est une vue de détail sur la vanne de vidange du bassin ;
- La figure 5 est une vue en perspective de l'ensemble avec le bassin raccordé à l'unité fonctionnelle placée à distance ;
- La figure 6 est une vue de dessus du bassin, couvercle ôté, dans lequel l'unité fonctionnelle est rangée.

### Description détaillée de modes de réalisation

Dans le mode de réalisation décrit ci-après, on fait référence à un ensemble de récupération musculaire par immersion partielle ou totale, destiné principalement à la cryothérapie. Cet exemple, non limitatif, est donné pour une meilleure compréhension de l'invention et n'exclut pas l'utilisation d'un tel ensemble pour d'autres traitements physiologiques par immersion, dans une eau froide, chaude ou contrastée (chaude - froide).

Dans la suite de la description, le terme « ensemble » désigne, sauf indication contraire, un ensemble de récupération musculaire par immersion selon l'invention, et le terme « groupe froid » désigne un système connu de régulation de la température d'un liquide aussi bien par refroidissement que par chauffage.

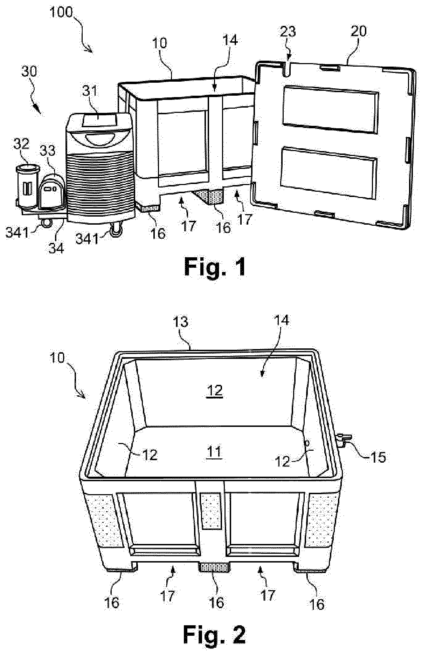

La figure 1 représente un ensemble 100 de récupération musculaire par immersion comprenant un bassin 10 destiné à être rempli d'un liquide de traitement tel que l'eau, un couvercle 20 pour fermer le bassin, une unité fonctionnelle 30 constituée d'un groupe froid 31 couplé à un dispositif de filtration 32 avec une pompe 33 en circulation externe, et optionnellement un chariot 34 mobile comportant des roues 341 et supportant ladite unité fonctionnelle. La présence du chariot 34 mobile facilite le déplacement de l'unité fonctionnelle 30.

L'ensemble 100 permet à un utilisateur, par exemple un sportif après un effort intense, d'immerger son corps, totalement ou partiellement, dans l'eau du bassin 10 préalablement rempli, celle-ci étant maintenue à une température favorisant une récupération ou une stimulation musculaire suivant un programme bien défini. Le maintien de l'eau du bassin 10 à des températures adéquates est assuré par l'unité fonctionnelle 30, et plus précisément par le groupe froid 31.

Le bassin 10, selon l'exemple de réalisation illustré, notamment sur la figure 2, présente une forme creuse globalement parallélépipédique à base rectangulaire, définissant un fond 11 et des parois latérales 12 surmontées d'un bord périphérique 13 et délimitant un volume intérieur 14 destiné à être rempli d'eau ou d'un autre liquide approprié.

Le fond 11 et les parois latérales 12 présentent intérieurement un état de surface lisse pour faciliter leur nettoyage et les rendre agréables au contact.

Extérieurement, le bassin 10 présente des arêtes verticales arrondies pour un meilleur agrément esthétique et comporte des évidements pour alléger son poids et des poignées pour être soulevé.

Le volume intérieur 14 peut recevoir une quantité d'eau plus ou moins importante selon les dimensions du bassin 10. Par ailleurs, le bassin 10 peut être individuel ou collectif, autrement dit il peut être dimensionné pour recevoir un seul utilisateur ou plusieurs utilisateurs à la fois. Bien entendu, cette distinction est basée sur une immersion totale du corps et un bassin individuel peut parfaitement être utilisé simultanément par plusieurs utilisateurs pour une immersion partielle (mollets, avant-bras, etc.).

Par exemple, le bassin 10 présente des dimensions extérieures d'environ 1200*1000*780mm, pour lesquelles le volume intérieur 14 se remplit en 30 minutes environ à débit courant.

Selon l'exemple illustré, la forme du bassin 10 rappelle celle d'une caisse-palette classique utilisée dans différentes industries pour transporter aussi bien un contenu solide qu'un contenu liquide. Ce format permet de faciliter le transport et la manipulation du bassin 10.

Alternativement, le bassin peut présenter une forme quelconque telle qu'une forme cylindrique à base circulaire, ovale ou polygonale, une forme pyramidale inversée, une forme en calotte sphérique ou toute autre forme définissant un volume intérieur suffisant pour l'immersion.

Le bord périphérique 13 est conformé pour coopérer avec le couvercle 20, par emboitement élastique par exemple.

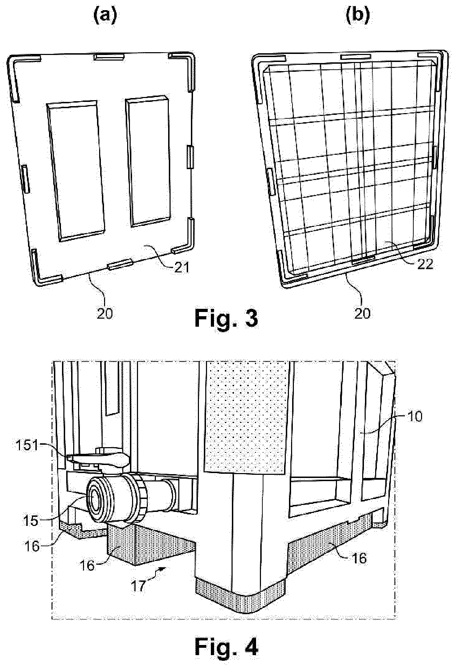

Le couvercle 20, selon l'exemple de réalisation illustré, notamment sur la figure 3, présente une forme rectangulaire arrondie aux sommets pour suivre les arrondis du bassin 10, définissant une face extérieure 21 visible en (a) et une face intérieure 22 visible en (b).

Le couvercle 20 est conçu de sorte à ce que sa tenue mécanique soit suffisante tout en restant léger pour faciliter son transport. A cet effet, le couvercle 20 comporte de nombreux évidements et des raidisseurs distribués comme sur l'exemple de la figure 3.

Le bassin 10 et le couvercle 20 peuvent également comporter des moyens de verrouillage pour bien sécuriser la fermeture si nécessaire. Cette fermeture peut éventuellement se faire de façon hermétique grâce à un joint d'étanchéité périphérique.

Ainsi, une fermeture sécurisée du bassin permettrait de contrôler tout accès audit bassin.

Le bassin 10 et le couvercle 20 sont fabriqués dans un matériau à la fois léger et résistant, qui soit également adapté à contenir des liquides à basse température, tel qu'une matière plastique, de préférence simple et peu couteuse à la fabrication.

Préférablement, le bassin 10 et le couvercle 20 sont fabriqués en polyéthylène, et plus préférablement en polyéthylène haute densité (PEHD), par un procédé de fabrication conventionnel tel que le moulage, la fabrication additive (impression 3D), etc.

Le bassin 10, selon l'exemple de réalisation illustré, comporte en outre une vanne 15 de vidange placée au niveau de la base dudit bassin, comme représenté sur la figure 4, et des semelles 16 au moyen desquelles il repose sur le sol.

La vanne 15 permet de vidanger l'eau du bassin 10 lorsqu'elle est ouverte, et donc de garder l'eau dans le bassin lorsqu'elle est fermée, et comprend un embout pour raccorder un tuyau et une poignée 151 d'ouverture et de fermeture.

La vanne 15 permet de déconnecter l'unité fonctionnelle 30 tout en conservant le liquide d'immersion dans le bassin 10.

Les semelles 16, selon l'exemple de réalisation illustré, sont au nombre de trois et s'étendent longitudinalement sous le bassin 10, entre deux parois latérales opposées, en étant sensiblement parallèles et espacées de sorte à définir des passages 17 empêchant la stagnation d'eau et autorisant un nettoyage sous le bassin 10 pour une meilleure hygiène. Les semelles 16 peuvent être antidérapantes pour améliorer l'adhérence du bassin 10 au sol, et éviter des accidents lors de l'entrée dans le bassin surtout lorsque le sol est humide.

Les semelles 16 permettent également de protéger le fond du bassin 10 lors de son transport en limitant les risques d'impacts et en évitant l'usure par frottement sur des surfaces à relief (fixations en saillie sur le plancher d'un véhicule utilitaire par exemple).

Les passages 17 permettent de pouvoir insérer entre les semelles 16 et sous le fond du bassin 10 les branches d'une fourche d'un transpalette.

Comme illustré sur les figures 1, 2 et 4, les parois latérales 12 comprennent des renforts verticaux positionnés notamment au niveau des arêtes verticales. En complément, les semelles 16 sont conçues pour s'insérer dans le bord périphérique 13. Ainsi, des bassins 10 peuvent être empilés les uns sur les autres.

Le bassin 10 peut être aisément raccordé à l'unité fonctionnelle 30 pour réfrigérer l'eau dans un circuit fermé, par des tuyaux d'arrivée et de départ d'eau, longs pour placer ladite unité à une certaine distance du bassin. De plus, l'unité fonctionnelle est amovible et peut être complétement séparée du bassin 10. Le fait que l'unité fonctionnelle 30 comprenant un groupe froid 31 et un dispositif de filtration 32 soit amovible et puisse être dissociée du bassin 10 permet de rendre ce dernier moins lourd et donc plus facilement transportable.

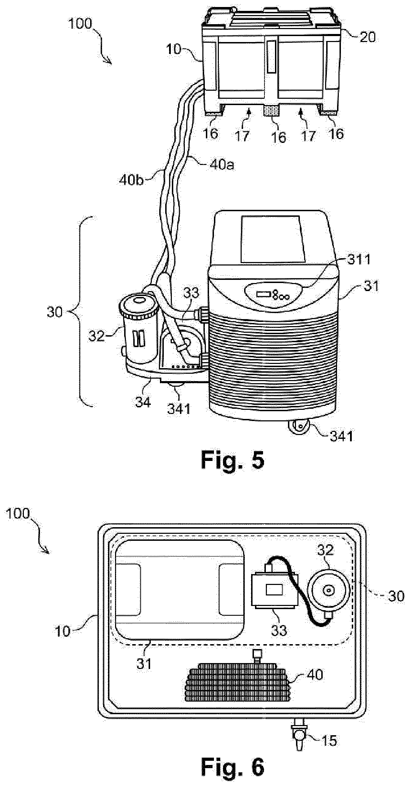

La figure 5 représente l'ensemble 100 avec le bassin 10 placé à distance suffisante de l'unité fonctionnelle 30 pour que celle-ci ne gêne pas les utilisateurs et puisse fonctionner de façon optimale en étant par exemple placée dans un local spécialement aménagé.

En effet, le groupe froid 31 doit être de préférence placé dans une pièce ventilée pour une évacuation efficace de la chaleur dégagée lors de son fonctionnement.

Cela est rendu possible grâce à des tuyaux 40a et 40b de longueur suffisante.

Par exemple, dans le cas d'une utilisation de l'ensemble 100 dans un vestiaire de club sportif, le bassin peut être placé dans la pièce principale du vestiaire pour une meilleure commodité, tandis que l'unité fonctionnelle 30 peut être placée dans un local technique.

Le groupe froid 31 permet de réfrigérer ou de chauffer l'eau à une température choisie qui peut être réglée via un panneau numérique de commande 311 permettant de contrôler un microprocesseur intégré.

Pour les besoins de l'invention, tout groupe froid adapté peut être utilisé, et notamment les groupes froids pour aquariums, largement disponibles dans le commerce.

Le groupe froid 31 se caractérise, entre autres, par sa capacité de réfrigération qui détermine le volume maximal d'eau pouvant être réfrigéré.

Si sa capacité de réfrigération le permet, le groupe froid 31 peut être raccordé à plusieurs bassins 10 en même temps. Il suffit pour ce faire de prévoir des tuyaux à plusieurs branches. Le groupe froid 31 est couplé au dispositif de filtration 32 car il est indispensable de filtrer l'eau avant de la faire circuler dans le groupe froid sinon l'évaporateur dudit groupe se colmatera et cela nuira à la réfrigération ou au chauffage.

Le dispositif de filtration 32 est un simple filtre à cartouche, de préférence à toile plissée en papier pour une filtration plus fine et un entretien facilité (lavage au jet d'eau et remplacement facile). Une filtration fine est nécessaire surtout lorsque les utilisateurs pratiquent un sport salissant qui se joue sur gazon par exemple (football, rugby, etc.), et seraient malencontreusement amenés à utiliser le bassin juste après un match sans se débarrasser des particules de terre accrochées à leur peau. Cette utilisation du bassin avant un lavage préalable des membres qui seront immergés est d'autant plus probable que les séances de récupération musculaire par cryothérapie sont recommandées immédiatement après l'effort (dans les quinze à vingt minutes qui suivent).

De plus, le bassin 10 peut comporter un préfiltre au niveau de la vanne 15 pour par exemple filtrer les particules les plus volumineuses préalablement à la filtration fine assurée par le dispositif de filtration 32 de l'unité fonctionnelle 30.

En fonctionnement, l'eau du bassin 10 est prélevée par la pompe 33 avant de traverser le filtre 32, amenée par le tuyau 40a raccordé à la vanne de vidange dudit bassin, et de pénétrer dans le groupe froid 31. Une eau à température régulée ressort du groupe froid 31 traverse le tuyau 40b avant d'être refoulée dans le bassin 10 par-dessus le bord périphérique 13 de ce dernier. A ce dernier effet, une entaille 23 peut être ménagée dans le couvercle 20 pour céder le passage à l'embout du tuyau 40b de refoulement comme représenté sur la figure 1, pour un fonctionnement avec le bassin 10 fermé.

Le tuyau de refoulement 40b peut également être placé loin du bassin 10 pour permettre de vidanger ce dernier loin de son espace d'utilisation. Idéalement, les tuyaux présentent des longueurs suffisantes pour permettre une vidange du bassin 10 à une distance au moins égale à 2m. La vidange peut se faire suivant le même circuit que lors du fonctionnement mais sans régulation de température.

La figure 6 représente le bassin 10 dans le volume intérieur duquel l'unité fonctionnelle 30 est entièrement rangée pour un transport optimisé de l'ensemble 100.

De préférence, le rangement de l'unité fonctionnelle 30 n'entrave pas la fermeture du bassin 10 avec le couvercle 20 pour un transport plus efficace. Ainsi, le bassin 10 est dimensionné de manière à contenir l'unité fonctionnelle 30 de réfrigération et/ou de chauffage (le groupe froid, le dispositif de filtration 32, la pompe 33 et le chariot 34 mobile) avec le couvercle 20 en position fermée. Le rangement de l'unité fonctionnelle 30 dans le bassin 10 permet d'obtenir un ensemble compact pour le stockage et le transport.

Bien entendu, certains éléments de l'ensemble de récupération musculaire par immersion peuvent être modifiés ou supprimés et des ajustements peuvent être apportés à son utilisation, sans pour autant sortir du cadre de l'invention, celui-ci étant défini par les revendications suivantes.

## Revendications

1. Ensemble (100) de récupération musculaire par immersion, comprenant un bassin (10) et une unité fonctionnelle (30) de réfrigération et/ou de chauffage d'un liquide contenu dans le bassin, ladite unité comprenant un groupe froid (31) couplé à un dispositif de filtration (32) et à une pompe (33) en circulation externe, ledit bassin se raccordant à ladite unité par des tuyaux (40), **caractérisé en ce que** l'ensemble est entièrement transportable et **en ce que** ledit bassin définit un volume intérieur (14) dans lequel l'unité fonctionnelle peut être rangée, le bassin (10) comportant à sa base des semelles (16) au nombre de trois qui s'étendent longitudinalement sous le bassin (10), entre deux parois latérales opposées, en étant sensiblement parallèles et espacées de sorte à définir des passages (17) empêchant la stagnation d'eau et autorisant un nettoyage sous le bassin (10) pour une meilleure hygiène, **en ce que** l'ensemble comprend en outre un couvercle (20) de fermeture du bassin (10), le bassin (10) étant dimensionné de manière à contenir l'unité fonctionnelle (30) de réfrigération et/ou de chauffage avec le couvercle (20) en position fermée, une entaille (23) étant ménagée dans le couvercle (20) pour céder le passage à un embout d'un tuyau (40b), pour un fonctionnement avec le bassin (10) fermé.

2. Ensemble selon l'une quelconque des revendications précédentes, dans lequel l'unité fonctionnelle (30) est montée sur un chariot mobile (34).

3. Ensemble selon l'une quelconque des revendications précédentes, dans lequel le bassin (10) est fabriqué en polyéthylène haute densité (PEHD).

4. Ensemble selon l'une quelconque des revendications précédentes, dans lequel le bassin (10) comporte une seule vanne (15) raccordée à l'unité fonctionnelle (30) par un premier tuyau (40a), et dans lequel un deuxième tuyau (40b) est raccordé à l'unité fonctionnelle et permet à la fois de refouler un liquide à température régulée dans le bassin et de vidanger ce dernier.

## Patentansprüche

1. Anordnung (100) zur Muskelregeneration durch Eintauchen, mit einem Becken (10) und einer funktionellen Einheit (30) zur Kühlung und/oder Heizung einer in dem Becken enthaltenen Flüssigkeit, wobei die Einheit ein Kühlaggregat (31) aufweist, das mit einer Filtervorrichtung (32) und einer Pumpe (33) in einem externen Kreislauf gekoppelt ist, und das Becken durch Schläuche (40) mit der Einheit verbunden ist, **dadurch gekennzeichnet, dass** die Anordnung vollständig transportabel ist, und dass das Becken ein Innenvolumen (14) bildet, in dem die funktionelle Einheit verstaubar ist, wobei das Becken (10) an seiner Basis drei Träger (16) aufweist, die sich in Längsrichtung unter dem Becken (10) zwischen zwei gegenüberliegenden Seitenwänden erstrecken, wobei sie im Wesentlichen parallel und beabstandet sind, um Durchgänge (17) zu bilden, die einen Wasserstau verhindern und eine Reinigung unter dem Bekken (10) für eine bessere Hygiene ermöglichen, und dass die Anordnung ferner einen Deckel (20) zum Verschließen des Bekkens (10) aufweist, wobei das Becken (10) so bemessen ist, dass es die funktionelle Einheit (30) zur Kühlung und/oder Heizung bei geschlossenem Deckel (20) aufnehmen kann, und wobei eine Aussparung (23) in dem Deckel (20) vorgesehen ist, um einem Endstück eines Schlauchs (40b) Durchgang zu gewähren, für einen Betrieb bei geschlossenem Becken (10).

2. Anordnung nach einem der vorhergehenden Ansprüche, wobei die funktionelle Einheit (30) auf einem fahrbaren Wagen (34) angeordnet ist.

3. Anordnung nach einem der vorhergehenden Ansprüche, wobei das Becken (10) aus hochdichtem Polyethylen (HDPE) gefertigt ist.

4. Anordnung nach einem der vorhergehenden Ansprüche, wobei das Becken (10) ein einzelnes Ventil (15) aufweist, das über einen ersten Schlauch (40a) mit der funktionellen Einheit (30) verbunden ist, und wobei ein zweiter Schlauch (40b) mit der funktionellen Einheit verbunden ist und dazu dient, sowohl eine temperierte Flüssigkeit in das Becken zurückzuführen als auch dieses zu entleeren.

## Claims

1. Assembly (100) for muscle recovery by immersion, comprising a tub (10) and a functional system (30) for refrigerating and/or heating a liquid contained in the tub, said system comprising a cold unit (31) coupled to a filtration device (32) and to a pump (33) in external circulation, said tub being connected to said system by pipes (40), **characterized in that** the assembly is entirely transportable and **in that** said tub defines an interior volume (14) in which the functional system can be stored, the tub (10) having, at its base, three soles (16) which extend longitudinally under the tub (10), between two opposite side walls, being substantially parallel and spaced apart so as to define passages (17) preventing the stagnation of water and allowing cleaning under the tub (10) for better hygiene, and **in that** the assembly further comprises a cover (20) for closing the tub (10), the tub (10) being dimensioned in such a way as to contain the refrigerating and/or heating functional system (30) with the cover (20) in the closed position, a notch (23) being formed in the cover (20) to allow an end of a pipe (40b) to pass through, for operation with the tub (10) closed.

2. Assembly according to any one of the preceding claims, in which the functional system (30) is mounted on a movable carriage (34).

3. Assembly according to any one of the preceding claims, in which the tub (10) is made of high-density polyethylene (HDPE).

4. Assembly according to any one of the preceding claims, in which the tub (10) has a single valve (15) connected to the functional system (30) by a first pipe (40a), and in which a second pipe (40b) is connected to the functional system and makes it possible both to deliver a temperature-controlled liquid into the tub and to drain the latter.
